# EUROPEAN PATENT APPLICATION

(11) **EP 2 229 944 A1**
(43) Date of publication of application: **22.09.2010**
(21) Application number: 08860372.5
(22) Date of filing: 11.12.2008
(51) Int. Cl.: A61K 31/427, A61K 9/08, A61K 9/14, A61K 47/04

(54) **PREPARATION COMPOSITION**

(30) Priority: 12.12.2007 JP 2007320852
(71) Applicant: Dainippon Sumitomo Pharma Co., Ltd., Osaka 541-8524 (JP)
(72) Inventor: NAKAGAWA, Takashi, Ibaraki-shi Osaka 567-0878 (JP)
(74) Representative: Vossius & Partner
(86) International application number: PCT/JP2008/072470
(87) International publication number: WO 2009/075309

(57) **Abstract**

Disclosed is a composition which is a stable aqueous carbapenem solution. The composition comprises (a) 1 part by weight of (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or a pharmaceutically acceptable salt thereof; and (b) 0.002 to 0.040 part by weight of sodium bicarbonate and/or 0.002 to 0.008 part by weight of sodium carbonate. Also disclosed is a composition for producing the above mentioned composition.

## Description

### TECHNICAL FIELD

The present invention relates to a preparation, namely an aqueous solution containing carbapenem and a composition for producing it.

### BACKGROUND OF ART

(4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid (hereinafter this compound may be abbreviated as Compound A) or a pharmaceutically acceptable salt thereof (hereinafter this compound or a pharmaceutically acceptable salt thereof may be abbreviated as Compound A or its salt) is described for example, in patent document 1 and shows excellent antibacterial activity. As carbapenem compounds are generally degraded in an aqueous solution to produce degradation, it is very important to prepare a stable aqueous solution of them.

Furthermore, with regard to freeze-dried formulations or powder-filled formulations, they need reconstitution procedure and if the stability after the procedure is higher, it can be possible to take much time after the procedure until administration of them to a patient, and therefore the burden to the medical practitioners is reduced. Therefore, it has been desired to prepare a composition which becomes a stable aqueous solution in case of dissolution.

Although sodium bicarbonate and sodium carbonate are included in the compositions which are disclosed in, for example, patent documents 2 and 3, the compounds contained in the compositions are different in chemical structure from Compound A. Furthermore, as carbonate ion binds nitrogen atom of piperidine ring of carbapenems, these carbapenems are stabilized.

Therefore, the compounds principally need equimolar amount of sodium bicarbonate or sodium carbonate. Amount of sodium bicarbonate or sodium carbonate is required at least about 20% by weight of a carbapen.em compound.

In addition, non-patent document 1 discloses an example wherein sodium carbonate is included in a composition containing meropenem, a kind of carbapenem compounds. However, as meropenem is different in chemical structure from Compound A used in the composition of the present invention, amount of sodium carbonate is required at least about 20% by weight of a carbapenem compound as well.

In addition, non-patent document 2 discloses an example wherein a relatively small amount of sodium bicarbonate is included in a composition containing imipenem having carbapenem structure.

However, imipenem has a characteristic moiety having iminomethylamino group in its side chain, but carbapenem compounds described in patent documents 2 and 3, meropenem described in non-patent document 1, and Compound A related to the present invention have a 5-membered ring with nitrogan atom in their side chains and, therefore imipenem is greatly different in chemical structure from the latter three compounds.
Patent document 1: WO 02/38564 gazette
Patent document 2: Japanese patent publication (Tokuhyohei) 11-509871
Patent document 3: Japanese patent publication (Tokuhyo) 2000-508343
Non-patent document 1: Package insert of Meropen® for infusion 0.25g and Meropen® for infusion 0.5g
Non-patent document 2: Package insert of Tienam® for infusion

### DISCLOSURE OF INVENTION

### Problem to be solved by invention

The problem to be solved is to provide an aqueous solution of Compound A or its salt and a composition for producing it, which have such effects as suppression of degradation, suppression of its content loss, suppression of colorization and so on.

### Means for solving the problem

The present inventors studied stability of Compound A and its salt in an aqueous solution, and found that by adding sodium bicarbonate or sodium carbonate thereto, suppression of production of by-products, suppression of its content loss, suppression of colorization and so on were attained.

The present invention relates to following aspects.
[1] A composition comprising (a) 1 part by weight of Compound A or a pharmaceutically acceptable salt thereof; and (b) 0.002 to 0.040 part by weight of sodium bicarbonate and/or 0.002 to 0.008 part by weight of sodium carbonate.
[2] The composition according to above item [1] comprising (a) 1 part by weight of Compound A or a pharmaceutically acceptable salt thereof; and (b) 0.002 to 0.040 part by weight of sodium bicarbonate.
[3] The composition according to above item [1] comprising (a) 1 part by weight of Compound A or a pharmaceutically acceptable salt thereof; and (b) 0.002 to 0.008 part by weight of sodium carbonate.
[4] The composition according to above item [2] wherein amount of sodium bicarbonate is 0.005 to 0.030 part by weight.
[5] The composition according to above item [3] wherein amount of sodium carbonate is 0.003 to 0.005 part by weight.
[6] The composition according to any one of above items [1] to [5] wherein the composition is a powder.
[7] The composition according to above item [6] wherein amount of Compound A or a pharmaceutically acceptable salt thereof is 50% w/w or more.
[8] The composition according to any one of above items [1] to [5] wherein the composition is an aqueous solution.
[9] A kit containing (a) a composition comprising 1 part by weight of Compound A or a pharmaceutically acceptable salt thereof; and (b) a composition comprising 0.002 to 0.040 part by weight of sodium bicarbonate and/or 0.002 to 0.008 part by weight of sodium carbonate.
[10] The kit according to above item [9] containing (a) the composition comprising 1 part by weight of Compound A or a pharmaceutically acceptable salt thereof; and (b) the composition comprising 0.002 to 0.040 part by weight of sodium bicarbonate.
[11] The kit according to above item [9] containing (a) the composition comprising 1 part by weight of Compound A or a pharmaceutically acceptable salt thereof; and (b) the composition comprising 0.002 to 0.008 part by weight of sodium carbonate.
[12] The kit according to above item [10] wherein amount of sodium bicarbonate is 0.005 to 0.030 part by weight.
[13] The kit according to above item [11] wherein amount of sodium carbonate is 0.003 to 0.005 part by weight.
[14] The kit according to any one of above items [9] to [13] wherein the composition (a) is a powder.
[15] The kit according to above item [14] wherein amount of Compound A or a pharmaceutically acceptable salt thereof in the composition (a) is 50% w/w or more.
[16] The kit according to any one of above item [9] to [13] wherein the composition (a) is an aqueous solution.
[17] A package of the kit containing (a) a composition comprising Compound A or a pharmaceutically acceptable salt thereof; and (b) an instruction indicating to dissolve the composition (a) by using an aqueous solution containing 0.002 to 0.040 part by weight of sodium bicarbonate and/or 0.002 to 0.008 part by weight of sodium carbonate per one part of Compound A or a pharmaceutically acceptable salt thereof.
[18] The package of the kit according to above item [17] containing (a) the composition comprising Compound A or a pharmaceutically acceptable salt thereof; and (b) instructions indicating to dissolve the composition (a) by using an aqueous solution containing 0.002 to 0.040 part by weight of sodium bicarbonate per one part of Compound A or a pharmaceutically acceptable salt thereof.
[19] The package of the kit according to above item [17] containing (a) the composition comprising Compound A or a pharmaceutically acceptable salt thereof; and (b) an instruction indicating to dissolve the composition (a) by using an aqueous solution containing 0.002 to 0.008 part by weight of sodium carbonate per one part of Compound A or a pharmaceutically acceptable salt thereof.
[20] The package of the kit according to above item [18] wherein amount of sodium bicarbonate is 0.005 to 0.030 part by weight.
[21] The package according to above item [18] wherein amount of sodium carbonate is 0.003 to 0.005 part by weight.
[22] The package of the kit according to any one of above item [17] to [21] wherein the composition (a) is a powder.
[23] The package of the kit according to above item [22] wherein amount of Compound A or a pharmaceutically acceptable salt thereof in the composition (a) is 50% w/w or more.

### Effect of the invention

According to the present invention, an aqueous solution of Compound A or its salt, and a composition for producing it, which have such characteristic properties as controls of degradation, suppression of its content loss, suppression of coloration and so on are provided by adding sodium bicarbonate and/or sodium carbonate thereto.

Amount of impurities contained in a medicine or a medical composition is strictly regulated and controlled by the Control Authorities. According to for example, the Guideline regarding to "impurities in New Drug Products" with attachment 1 put on http://www.pmda.go.jp/ich/quality.htm, when maximum daily dose is beyond 1g, threshold amount of impurity which has to reported is 0.05%, and when maximum daily dose is beyond 2g, threshold amount of impurity which has to determine its structure is 0.10% and when maximum amount of dosage per day is beyond 2g, threshold amount of impurity which has to confirm the safety is 0.15%.

Therefore, it can be recognized that the stability after dissolution has to be considered even on a solid preparation which is dissolved before using as well as an aqueous solution and that the preparations which produce as little impurities as possible are expected.

The present inventors found as follows:
When Compound A was preserved in an aqueous solution, productions of Compound B and Compound C which are described below and coloration were remarkable. Furthermore, production of Compound C and coloration were suppressed by adding a very small amount of sodium bicarbonate. When amount of it is further increased, production of Compound C and coloration were still suppressed, but another compound, namely Compound B was newly produced to reduce the content of Compound A.

Such effects as suppression of production of by-products, suppression of the content loss of Compound A, suppression of coloration and so on by adding a specific compound only in a small amount and within a narrower range are neither described nor suggested in above any documents.

Furthermore, the present inventors found that even if sodium carbonate was added thereto, the same effect was obtained, but optimal range of contents of sodium carbonate which suppresses production of by-products, suppresses the content loss, and suppresses coloration and so on, was narrower than that of sodium bicarbonate.

Such effects are neither described nor suggested in above any document, too.

Although the stabilized preparations containing carbapenems are described in the above patent documents 1 and 2, there is therein a description that a specific carbapenem reacts with a carbon dioxide source on nitrogen atom of the carbapenem as a stabilization factor. Therefore, according to these documents, it is necessary for the carbon dioxide source of an equimolecular or more to stabilize the carbapenem compound. In consideration that molecular weights of them are about 300 to 400 and molecular weights of sodium bicarbonate and sodium carbonate are 84 and 106, respectively, at least about 20%w/w of these carbonates should need. According to examples disclosed in these documents more than 20% w/w of sodium bicarbonate is used therein in fact.

On the contrary, regardless amounts of them used in the present invention are far smaller than one in the references, above desired effects and controls of coloration of a solution containing Compound A are attained.

### BRIEF DESCRIPTION OF DRAWINGS

Figure 1 corresponds to Table 12 and shows change of absorbance on a formulation containing Compound A/ sodium bicarbonate after dissolving it in physiological saline which solution is preserved at 25°C.
Figure 2 corresponds to Table 13 and shows change of absorbance on a formulation containing imipenem/ sodium bicarbonate after dissolving it in physiological saline which solution is preserved at 25°C.
Figure 3 corresponds to Table 15 and shows change of absorbance on a formulation containing Compound A/ sodium carbonate after dissolving it in physiological saline which solution is preserved at 25°C.
Figure 4 corresponds to Table 16 and shows change of absorbance on a formulation containing imipenem/sodium carbonate after dissolving it in a physiological saline which solution is preserved at 25°C.

### BEST MODE FOR CARRYING OUT THE INVENTION

The present invention is explained in more detail. (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}-sulfanyl)-7-oxo-1-azabicyclo[3.2.0]hept-2-ene-2-carboxylic acid is presented by the following chemical formula.

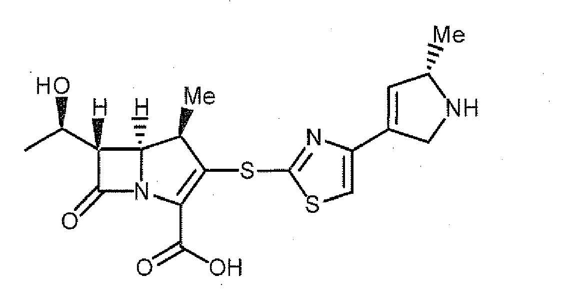

Pharmaceutical acceptable salts of Compound A include alkali metal salts such as sodium salt or potassium salt, alkaline earth metal salts such as calcium salt or magnesium salt, salts with an inorganic base such as ammonium salt, salts with an organic base such as triethylammonium salt, pyridinium salt, or diisopropylammonium salt, respectively formed with the carboxyl group, or an ammonium salt formed with (5S)-5-methyl-2,5-dihydro-1H-pyrrol-3-yl group of the above formula. Salts formed with an intramolecular basic group include salts formed with inorganic acids such as hydrochloric acid, sufuric acid, phosphoric acid, or organic acid such as formic acid, acetic acid, oxalic acid, methansulfonic acid, benzenesulfonic acid.

Amount of sodium bicarbonate is 0.002 to 0.040 part by weight, preferably 0.005 to 0.030 part by weight, and more preferably 0.008 to 0.024 part by weight per one part by weight of Compound A.

Amount of sodium carbonate is 0.002 to 0.008 part by weight, preferably 0.003 to 0.005 part by weight, and more preferably 0.003 to 0.0045 part by weight per one part by weight of Compound A.

The composition of the present invention can be prepared for example, following methods.

### (1) Solid formulation

For example, a powder-filled formulation is prepared by mixing Compound A or its salt, powdered sodium bicarbonate and/or sodium carbonate, and if necessary, a powdered excipient at their solid states. The mixing order of each ingredient is not limited. When a small amount of the ingredient is included, the appropriate methods are applied such as in a trituration, and so on.

A preparation for injection, for example, can be prepared by crystallization etc. after filter sterilization under aseptic condition, or obtaining each aseptic ingredient by radiation of x-ray or electron beam, and then filling the ingredient into a vial previously sterilized under aseptic condition, capped with a rubber stopper previously sterilized and sealed to produce the preparation for injection.

In case of a freeze-dried formulation, after dissolving in water a powdered mixture obtained as well and filter sterilization, the solution was filled into a vial previously sterilized under aseptic condition, lyophilized, capped with a stopper previously sterilized and sealed to produce the freeze-dried formulation.

A solution before lyophilization may be prepared by appropriately combining such methods as mixing each solutions after each ingredients are dissolved in water instead of dissolving the mixed powder in water, and so on.

### (2) Solution formulation

The formulation is prepared by dissolving or dispersing powdered Compound A or its salt, powdered sodium bicarbonate and/or sodium carbonate, and if necessary an excipient in a solvent such as water or a physiological saline, and then filling it into a vial and so on. The dissolving order of each ingredient is not limited.

In case of a preparation for injection, for example, after filter sterilization by filtration under aseptic condition, the mixture is filled into a vial previously sterilized under aseptic condition, capped with a stopper previously sterilized, and sealed to prepare the formulation for injection.

### (3) Kit and package of the kit

A kit is for example, prepared by mixing powdered Compound A or its salt and if necessary a powdered excipient in their solid states.

In case of a formulation for an injection, a kit can be, for example, prepared by the following methods: (1) After filter sterilization under aseptic condition, obtaining each aseptic ingredient by crystallization and so on, and each ingredient in powder is filled into a vial previously sterilized under aseptic condition, capped with a stopper previously sterilized, and sealed.
(2) Separately after preparing a composition containing sodium bicarbonate and/or sodium carbonate, preferably an aqueous solution, it is filled into a vial, an ampoule, a bag or a prefiled syringe.
(3) Each aseptic ingredient prepared in above (1) in powder is filled into one portion of a double bag previously sterilized under aseptic condition, and the solution prepared in above (2) is filled into the other portion of the double bag and then the bag is sealed.
(4) A vial prepared in above (1) and a vial, an ampoule, a bag or a prefiled syringe prepared in above (2) are packaged as a kit, or a vial prepared in above (1) is packed with a package insert indicating to dissolve it with a solution (2) commercialized.

The excipient includes fillers such as D-mannitol or D-sorbitol, and tonicity agents such as sodium chloride or dextrose. However, when amount of the excipient is much, as there is a possibility to affect the effect by sodium bicarbonate and /or sodium carbonate, amount of Compound A, carbapenem is 50% or more, preferably 80% or more, more preferably 90% or more, and the most preferably 95% or more per total amount of the composition provided that a tonicity agent such as sodium chloride, or dextrose is not included.

When the composition described in above item [1] is a powdered mixture, and the composition (a) in a kit described in above item [9] is a powder or Composition (a) in a package of the kit described in above item [17] is a powder, amount of Compound A or its pharmaceutically acceptable salt is 50% w/w or more, preferably 80% w/w or more, more preferably 90% w/w or more, and the most preferably 95% w/w or more.

In case of a solution formulation and a solution after reconstitution of a solid formulation such as a powder-filled formulation or a freeze-dried formulation, pH of the solution is preferably 6.0 to 7.4, more preferably 6.5 to 7.2, and the most preferably 6.7 to 7.1. However it should be considered that pH values measured have deviation error within error about 0.1

### Example

The composition of the present invention is explained in detail by the following examples and test examples, but is not limited by them.

### Example 1

Each ingredient of composition numbers 1 to 14 indicated in Tables 1 to 2 was mixed in the prescribed amount per vial, the mixture is sterilized by filtration, and filled into a vial previously sterilized, by weighing the prescribed amount, followed by capping with a rubber stopper previously sterilized and sealing with a flip-off cap, to produce a composition for injection.

**Table 1**

| Composition number | 1 | 2 | 3 | 4 | 5 | 6 | 7 | 8 |
|---|---|---|---|---|---|---|---|---|
| Compound A (mg) | 500 | 500 | 500 | 500 | 250 | 250 | 250 | 250 |
| sodium bicarbonate (mg) | 2.5 | 5.0 | 10 | 15 | 1.3 | 2.5 | 5 | 7.5 |
| physiological saline (ml, totally) | 100 | 100 | 100 | 100 | 100 | 100 | 100 | 100 |

**Table 2**

| Composition number | 9 | 10 | 11 | 12 | 13 | 14 |
|---|---|---|---|---|---|---|
| Compound A (mg) | 500 | 500 | 500 | 250 | 250 | 250 |
| sodium carbonate (mg) | 1.5 | 2.0 | 2.5 | 0.8 | 1.0 | 1.2 |
| physiological saline (ml, totally) | 100 | 100 | 100 | 100 | 100 | 100 |

### Example 2

Each ingredient of composition numbers 15 to 28 indicated in Tables 3 to 4 was mixed in the prescribed amount and the mixture was sterilized by filtration, filled into a vial previously sterilized by weighing the prescribed amount, followed by capping with a rubber stopper previously sterilized, and sealing with a flip-off cap, to produce a composition for injection which was dissolved before using.

**Table 3**

| Composition number | 15 | 16 | 17 | 18 | 19 | 20 | 21 | 22 |
|---|---|---|---|---|---|---|---|---|
| Compound A (mg) | 500 | 500 | 500 | 500 | 250 | 250 | 250 | 250 |
| sodium bicarbonate (mg) | 2.5 | 5.0 | 10 | 15 | 1.3 | 2.5 | 5 | 7.5 |

**Table 4**

| Composition number | 23 | 24 | 25 | 26 | 27 | 28 |
|---|---|---|---|---|---|---|
| Compound A (mg) | 500 | 500 | 500 | 250 | 250 | 250 |
| sodium carbonate (mg) | 1.5 | 2.0 | 2.5 | 0.8 | 1.0 | 1.2 |

### Test example 1

### Stability in an aqueous solution of a formulation containing sodium bicarbonate

Multiple vials were prepared and amounts of Compound A and sodium bicarbonate indicated in Table 5 were weighed and filled in each vial. Thereto was added physiological saline (100ml). Amounts of Compound A and its degradation, Compounds B and C were measured just after dissolution and after preservation for 6 hours at 25°C.

**Table 5**

| Compound A (g/vial) | sodium bicarbonate (mg/vial) |
|---|---|
| 0.5 | 0.0 |
| | 1.0 (0.002 part by weight) |
| | 2.5 (0.005 part by weight) |
| | 5.0 (0.01 part by weight) |
| | 10 (0.02 part by weight) |
| | 15 (0.03 part by weight) |
| | 20 (0.04 part by weight) |
| | 50 (0.1 part by weight) |
| | 100 (0.2 part by weight) |

Conditions of HPLC measurement;
column: SUMIPAX ODS-A212 (silica gel binding with octadecyl group, particle size: 5µm; 6.0mmϕ x 15cm)
detector: ultraviolet spectrophotometer (wavelength: 220nm)
column temperature: constant temperature around at 25°C
mobile phase: A solution; 5mmol/L phosphate buffer (pH7) and acetonitrile (300 : 23), B solution: acetonitrile
flow rate: 1.0mL/min.
gradient condition: as shown in Table 6

**Table 6**

| | | | | | | |
|---|---|---|---|---|---|---|
| Time (min.) | 0 | 30 | 60 | 70 | 70.1 | 90 |
| Mobile phase: B sol. (%) | 0 | 0 | 36 | 78 | 0 | 0 |

Compound A was detected at retention time; about 35 minutes under above condition of HPLC.
Compound B was detected at retention time; about 7 minutes under above condition of HPLC. LC-MS (m/z): 425
Compound C was detected at retention time: about 44 minutes under above condition of HPLC. LC-MS (m/z): 815
Condition of LC-MS measurement on Compounds B and C;
instrument: esquire 3000 plus (Bruker (USA))
HPLC condition;
column: SUMIPAX ODS-A212 (silica gel binding with octadecyl group, particle size: 5µm, 6.0mmϕ x 15cm)
detector: ultraviolet spectrophotometer (wavelength: 220nm)
column temperature: constant temperature around at 25°C
mobile phase: A solution: 1mmol/L ammonium acetate buffer (pH7) and acetonitrile (300 : 23) B solution: acetonitrile
flow rate: 1.0mL/mini.
gradient condition: as shown in following Table 7

**Table 7**

| | | | | | | |
|---|---|---|---|---|---|---|
| Time(min.) | 0 | 30 | 60 | 70 | 70.1 | 90 |
| Mobile phase B sol. (%) | 0 | 0 | 36 | 78 | 0 | 0 |

The results are shown in Table 8. The numbers described in columns of "just after dissolution" and "after preservation for 6 hours at 25°C" on Table 8 show area % per total peaks which appear until 55 minutes under the above HPLC conditions.

As shown in Table 8, it was found that when sodium bicarbonate was not added, production of Compound C was much remarkable. On the other hand, when 1mg of sodium bicarbonate was added to 500mg of Compound A, the production of Compound C was considerably suppressed, and when 2.5mg of sodium bicarbonate was added to 500mg of Compound A, the production of Compound C was 0.1% or less.

It was found that when amounts of sodium bicarbonate were further relatively increased, the production of Compound C was still suppressed, but on the contrary the production of Compound B was remarkably increased. Such increase of by-products gave an influence to reduction of amount of Compound A. When amounts of sodium bicarbonate was increased to 20mg from 15mg, reduction of amount of Compound A was 0.2%, but when amount of sodium bicarbonate was increased to 50mg from 20mg, the amount of Compound A was reduced to 1.7%.

As such it is neither described nor suggested in the above documents that there is an optimum range among amounts of additions to give an influence to suppression of production of by-products and suppression of content loss of Compound A.

**Table 8**

| Comp. A (g/vial) | sodiumbicarbonate (mg/vial) | just after dissolution | | | after preservation for 6 hrs. at 25°C | | |
|---|---|---|---|---|---|---|---|
| | | Comp.A | Comp. B | Comp. C | Comp. A | Comp. B | Comp. C |
| 0.5 | 0.0 | 98.8 | 0.7 | 0.0 | 95.8 | 3.0 | 0.5 |
| | 1.0 | 98.9 | 0.7 | 0.0 | 96.6 | 2.7 | 0.2 |
| | 2.5 | 98.9 | 0.7 | 0.0 | 96.6 | 2.8 | 0.0 |
| | 5.0 | 98.9 | 0.6 | 0.0 | 96.3 | 3.0 | 0.0 |
| | 10 | 98.9 | 0.7 | 0.0 | 95.9 | 3.5 | 0.0 |
| | 15 | 98.9 | 0.6 | 0.0 | 95.4 | 3.9 | 0.0 |
| | 20 | 98.8 | 0.6 | 0.0 | 95.2 | 4.1 | 0.0 |
| | 50 | 98.6 | 0.7 | 0.0 | 93.5 | 5.5 | 0.0 |
| | 100 | 98.9 | 0.6 | 0.0 | 92.4 | 6.6 | 0.0 |

### Test example 2

### Stability in an aqueous solution formulation containing sodium carbonate

Multiple vials were prepared, and Compound A and sodium carbonate in amounts indicated in Table 9 were weighed and put in each vial. A physiological saline (100ml) was added to each vial, amounts Compound A and its degradation, Compounds B and C were measured just after dissolving and after preserving for 6 hours at 25°C.
HPLC was measured under the same conditions as Test example 1.

**Table 9**

| Compound A (g/vial) | sodium carbonate (mg/vial) |
|---|---|
| 0.5 | 0.0 |
| | 1.0 (0.002 part by weight) |
| | 1.5 (0.003 part by weight) |
| | 2.0 (0.004 part by weight) |
| | 2.5 (0.005 part by weight) |
| | 3.0 (0.006 part by weight) |
| | 3.5 (0.007 part by weight) |
| | 4.0 (0.008 part by weight) |
| | 5.0 (0.01 part by weight) |

The results are shown in Table 10. The numbers described in columns of "just after dissolution" and "after preservation for 6 hours at 25°C" on Table 10 show area % per total peaks which appear until 55 minutes under the above HPLC conditions.

As shown in Table 10, it was found that the same tendency as in case of sodium bicarbonate was observed, but optimum range to show remarkable effect was further narrower. Namely when 1mg of sodium carbonate was added to 500mg of Compound A, production of Compound C was considerably suppressed, and when 1.5mg of sodium carbonate was added to 500mg of Compound A, production of Compound C was 0.1% or less.

It was found that when amounts of sodium carbonate were further relatively increased, production of Compound C was still suppressed, but on the contrary production of Compound B was increased. Namely when amount of sodium carbonate was increased to 5mg from 2mg, reduction of amount of Compound A was 1.4%.

Also in case of sodium carbonate, it is neither described nor suggested in the above documents that there is a remarkable optimum range among amounts of additions to give an influence to suppression of production of by-products and suppression of content loss of Compound A and especially further narrower range than in case of sodium bicarbonate.

**Table 10**

| Comp. A (g/vial) | sodium carbonate (mg/vial) | just after dissolution | | | after preservation for 6 hrs. 25°C | | |
|---|---|---|---|---|---|---|---|
| | | Comp. A | Comp. B | Comp. C | Comp. A | Comp. B | Comp. C |
| 0.5 | 0.0 | 99.0 | 0.5 | 0.0 | 96.3 | 2.6 | 0.4 |
| | 1.0 | 99.0 | 0.6 | 0.0 | 96.7 | 2.6 | 0.2 |
| | 1.5 | 98.9 | 0.5 | 0.0 | 96.5 | 2.8 | 0.0 |
| | 2.0 | 99.0 | 0.5 | 0.0 | 96.2 | 3.0 | 0.0 |
| | 2.5 | 98.9 | 0.5 | 0.0 | 96.0 | 3.2 | 0.0 |
| | 3.0 | 99.0 | 0.5 | 0.0 | 95.7 | 3.6 | 0.0 |
| | 3.5 | 98.9 | 0.5 | 0.0 | 95.4 | 3.8 | 0.0 |
| | 4.0 | 99.0 | 0.5 | 0.0 | 95.1 | 4.1 | 0.0 |
| | 5.0 | 98.9 | 0.6 | 0.0 | 94.8 | 4.5 | 0.0 |

### Example 3

### Evaluation on control of coloration in an aqueous solution formulation containing sodium bicarbonate in water

Multiple vials were prepared, and Compound A, imipenem and sodium bicarbonate in amounts indicated in Table 11 were weighed, respectively and each mixture was put in each vial. A physiological saline (100ml) was added to each vial to be dissolved. In addition, to a vial containing Tienam® for infusion (0.5g potency/vial) was added a physiological saline (100ml). Coloration (change of color) just after dissolution and when the solution was preserved at one hour intervals for 6 hours at 25°C were measured by absorbency.

**Table 11**

| sodium bicarbonate (mg/vial) | Compound A (g/vial) | imipenem (g/vial) |
|---|---|---|
| 0.0 | 0.5 | 0.5 |
| 1.0 (0.002 part by weight) | | |
| 2.5 (0.005 part by weight) | | |
| 5.0 (0.01 part by weight) | | |
| 10 (0.02 part by weight) | | |
| 20 (0.04 part by weight) | | |
| 50 (0.1 part by weight) | | |

Conditions of measurement by spectrophotometer;
cell: quartz 12.5mm x 12.5mm x 45mm, capacity 3.5ml
wavelength: 380nm
measurement temperature: 25°C
instrument: UV-2200A (Shimadzu)

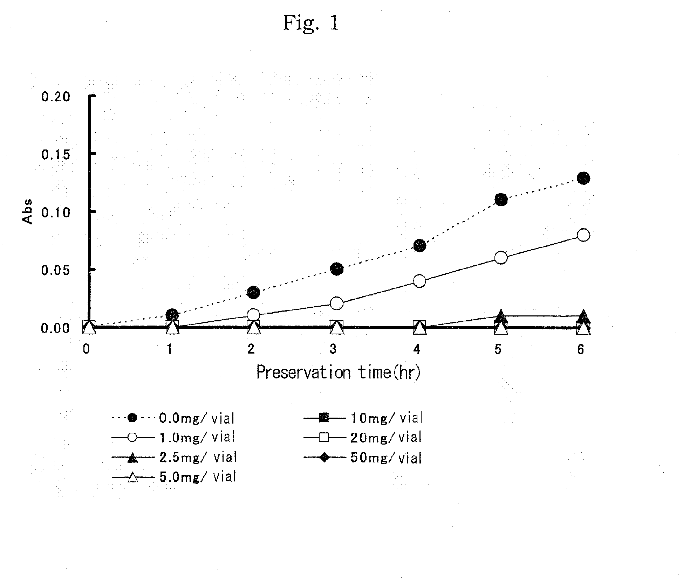

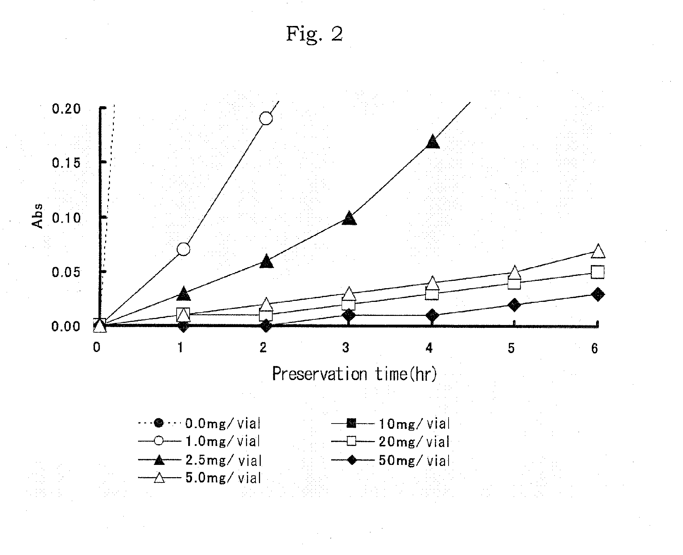

The results are shown in Table 12, Figures 1 and 2.

The numbers described in columns of "just after dissolution" and each "preservation time" on Table 12 show values of change in absorbance just after dissolution when measured under the above conditions.

As shown in Table 12, after preservation of Compound A for 6 hours when sodium bicarbonate was not added, values of change in absorbance was 0.13, that means remarkable coloration was detected. On the other hand, when sodium bicarbonate (1.0mg) was added per Compound A (500mg), values of change in absorbance was 0.08, that means the tendency that coloration was suppressed was observed. When sodium bicarbonate .(2.5mg) was added per Compound A (500mg), values of change in absorbance was 0.01, and coloration was not observed by the visual inspection when compared with a solution just after dissolution.

**Table 12**

| Comp. A (g/vial) | sodium bicarbonate (mg/vial) | preservation time (hr) | | | | | | |
|---|---|---|---|---|---|---|---|---|
| | | just after dissolution | 1 | 2 | 3 | 4 | 5 | 6 |
| | 0.0 | 0.00 | 0.01 | 0.03 | 0.05 | 0.07 | 0.11 | 0.13 |
| | 1.0 | 0.00 | 0.00 | 0.01 | 0.02 | 0.04 | 0.06 | 0.08 |
| | 2.5 | 0.00 | 0.00 | 0.00 | 0.00 | 0.00 | 0.01 | 0.01 |
| 0.5 | 5.0 | 0.00 | 0.00 | 0.00 | 0.00 | 0.00 | 0.00 | 0.00 |
| | 10 | 0.00 | 0.00 | 0.00 | 0.00 | 0.00 | 0.00 | 0.00 |
| | 20 | 0.00 | 0.00 | 0.00 | 0.00 | 0.00 | 0.00 | 0.00 |
| | 50 | 0.00 | 0.00 | 0.00 | 0.00 | 0.00 | 0.00 | 0.00 |

| | | | | | | | | |
|---|---|---|---|---|---|---|---|---|
| Unit: Abs | | | | | | | | |

As shown in Table 13, when sodium bicarbonate was not added after preservation of imipenem for 6 hours, values of change in absorbance was 4.65 and remarkable coloration was detected. On the other hand, when 1.0mg and 2.5mg of sodium bicarbonate were respectively added per imipenem (500mg), values of change in absorbance were 0.42 and 0.38, respectively and therefore suppression of coloration was not observed. Furthermore, when 5.0mg, 10mg and 20mg of sodium bicarbonate were respectively added per imipenem (500mg), and when Tienam® for infusion (0.5g potency/vial, sodium carbonate 20mg/vial, cilastatin sodium 0.5g/vial) was used, their values of change in absorbance were range of 0.05 to 0.07, and coloration was observed by the visual inspection. On the other hand, when sodium bicarbonate (50mg) was added per imipenem (500mg), values of change in absorbance were 0.03, and the tendency that coloration was suppressed was observed, but suppression of coloration was still not sufficient when compared with Compound A.

**Table 13**

| imipenem (g/vial) | sodium bicarbonate (mg/vial) | preservation time (hr) | | | | | | |
|---|---|---|---|---|---|---|---|---|
| | | just after dissolution | 1 | 2 | 3 | 4 | 5 | 6 |
| 0.5 | 0.0 | 0.00 | 1.12 | 3.42 | 3.99 | 4.47 | 4.47 | 4.65 |
| | 1.0 | 0.00 | 0.07 | 0.19 | 0.30 | 0.39 | 0.41 | 0.42 |
| | 2.5 | 0.00 | 0.03 | 0.06 | 0.10 | 0.17 | 0.25 | 0.38 |
| | 5.0 | 0.00 | 0.01 | 0.02 | 0.03 | 0.04 | 0.05 | 0.07 |
| | 10 | 0.00 | 0.01 | 0.01 | 0.02 | 0.03 | 0.04 | 0.05 |
| | 20 | 0.00 | 0.01 | 0.01 | 0.02 | 0.03 | 0.04 | 0.05 |
| | 50 | 0.00 | 0.00 | 0.00 | 0.01 | 0.01 | 0.02 | 0.03 |
| Tienam for diffusion (0.5g potency) | | 0.00 | 0.01 | 0.01 | 0.02 | 0.03 | 0.04 | 0.05 |

| | | | | | | | | |
|---|---|---|---|---|---|---|---|---|
| Unit: Abs | | | | | | | | |

When at least 2.5mg (0.005 part by weight) of sodium bicarbonate was added to 500mg of Compound A, suppression of coloration of the solution was observed.

### Example 4

### Evaluation on control of coloration in an aqueous solution formulation containing sodium carbonate in water

Multiple vials were prepared, and sodium carbonate, Compound A and imipenem in amounts indicated in Table 14 were weighed, respectively and each mixture was put in each vial. A physiological saline (100ml) was added to each vial to be dissolved. Coloration (change of color) just after dissolution and when the solution was preserved at one hour intervals for 6 hours at 25°C were measured by absorbency on Compound A and imipenem.

**Table 14**

| sodium carbonate (mg/vial) | Compound A (g/vial) | imipenem (g/vial) |
|---|---|---|
| 0.0 | 0.5 | 0.5 |
| 1.0 (0.002 part by weight) | | |
| 1.5 (0.003 part by weight) | | |
| 2.0 (0.004 part by weight) | | |
| 2.5 (0.005 part by weight) | | |
| 3.0 (0.006 part by weight) | | |

Condition of measurement by spectrophotometer
cell: quartz 12.5mm x 12.5mm x 45mm, capacity 3.5ml
wavelength: 380nm
measurement temperature: 25°C
instrument: UV-2200A (Shimadzu)

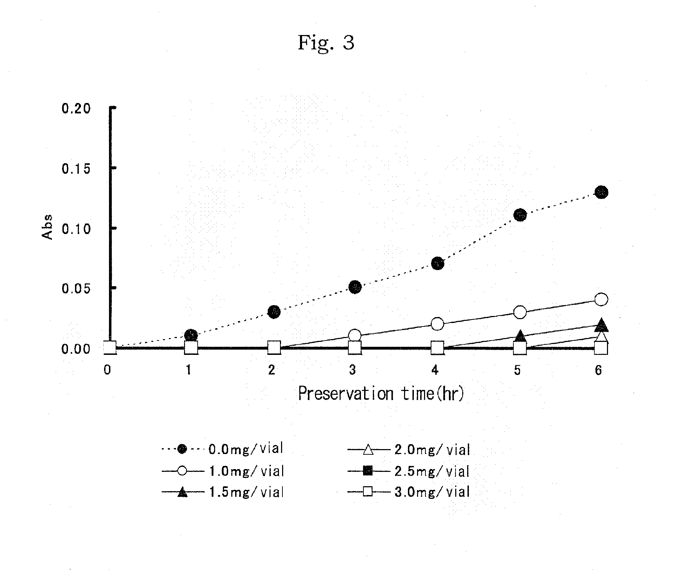

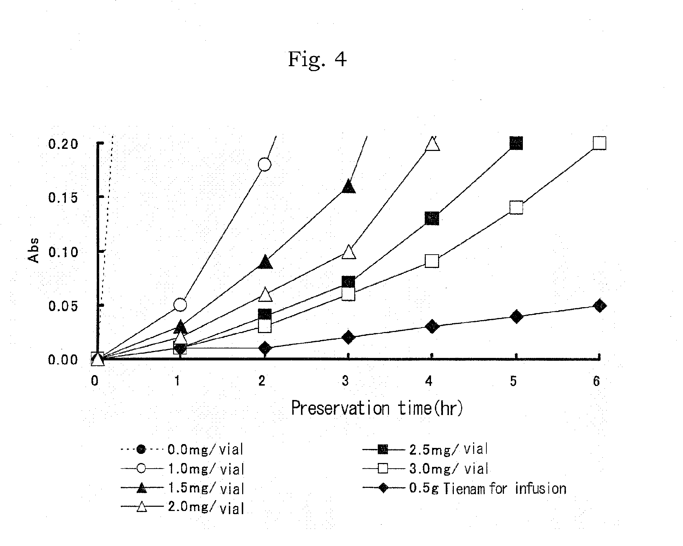

The results are shown in Table 15, Figures3 and 4.

The numbers described in columns of "just after dissolution" and each "preservation time" on Table 15 show values of change in absorbance just after dissolution when measured under the above conditions.

As shown in Table 15, when sodium carbonate was not added after preservation of Compound A for 6 hours, values of change in absorbance was 0.13 and remarkable coloration was detected. On the other hand, when sodium carbonate (1.0mg) was added per Compound A (500mg), values of change in absorbance was 0.04, that means the tendency that coloration was suppressed was observed. When sodium bicarbonate (1.5mg) was added per Compound A

(500mg), values of change in absorbance was 0.02 and coloration was not observed by the visual inspection when compared with a solution just after dissolution.

**Table 15**

| Comp. A (g/vial) | sodium carbonate (mg/vial) | preservation time (hr) | | | | | | |
|---|---|---|---|---|---|---|---|---|
| | | just after dissolution | 1 | 2 | 3 | 4 | 5 | 6 |
| 0.5 | 0.0 | 0.00 | 0.01 | 0.03 | 0.05 | 0.07 | 0.11 | 0.13 |
| | 1.0 | 0.00 | 0.00 | 0.00 | 0.01 | 0.02 | 0.03 | 0.04 |
| | 1.5 | 0.00 | 0.00 | 0.00 | 0.00 | 0.00 | 0.01 | 0.02 |
| | 2.0 | 0.00 | 0.00 | 0.00 | 0.00 | 0.00 | 0.00 | 0.01 |
| | 2.5 | 0.00 | 0.00 | 0.00 | 0.00 | 0.00 | 0.00 | 0.00 |
| | 3.0 | 0.00 | 0.00 | 0.00 | 0.00 | 0.00 | 0.00 | 0.00 |

| | | | | | | | | |
|---|---|---|---|---|---|---|---|---|
| Unit: Abs | | | | | | | | |

As shown in Table 16, it was found remarkable coloration that values of change in absorbance was 4.65 when sodium carbonate was not added after preservation of imipenem for 6 hours. Furthermore, when 1.0mg, 1.5mg, 2.0mg, 2.5mg and 3.0mg of sodium carbonate were respectively added per imipenem (500mg), values of change in absorbance were 0.20 to 3.44 and therefore suppression of coloration was not observed.

**Table 16**

| imipenem (g/vial) | sodium carbonate (mg/vial) | preservation time (hr) | | | | | | |
|---|---|---|---|---|---|---|---|---|
| | | just after dissolution | 1 | 2 | 3 | 4 | 5 | 6 |
| 0.5 | 0.0 | 0.00 | 1.12 | 3.42 | 3.99 | 4.47 | 4.47 | 4.65 |
| | 1.0 | 0.00 | 0.05 | 0.18 | 0.38 | 1.00 | 2.08 | 3.44 |
| | 1.5 | 0.00 | 0.03 | 0.09 | 0.16 | 0.37 | 0.75 | 1.39 |
| | 2.0 | 0.00 | 0.02 | 0.06 | 0.10 | 0.20 | 0.35 | 0.58 |
| | 2.5 | 0.00 | 0.01 | 0.04 | 0.07 | 0.13 | 0.20 | 0.30 |
| | 3.0 | 0.00 | 0.01 | 0.03 | 0.06 | 0.09 | 0.14 | 0.20 |

| | | | | | | | | |
|---|---|---|---|---|---|---|---|---|
| Unit: Abs | | | | | | | | |

When at least 1.5mg (0.003 part by weight) of sodium carbonate was added to 500mg of Compound A, suppression of coloration of the solution was observed.

### INDUSTRIAL APPLICABILITY

The present invention provides an aqueous solution containing Compound A or its salt, and a composition for producing it which have characteristic properties such as suppression of production of by-products, suppression of the content loss of Compound A and suppression of coloration.

## Claims

1. A composition comprising (a) 1 part by weight of (4R,5S,6S)-6-[(1R)-1-hydroxyethyl]-4-methyl-3-({4-1(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or a pharmaceutically acceptable salt thereof; and (b) 0.002 to 0.040 part by weight of sodium bicarbonate and/or 0.002 to 0.008 part by weight of sodium carbonate.

2. The composition according to claim 1 comprising (a) 1 part by weight of (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or a pharmaceutically acceptable salt thereof; and (b) 0.002 to 0.040 part by weight of sodium bicarbonate.

3. The composition according to claim 1 comprising (a) 1 part by weight of (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or a pharmaceutically acceptable salt thereof; and (b) 0.002 to 0.008 part by weight of sodium carbonate.

4. The composition according to claim 2 wherein amount of sodium bicarbonate is 0.005 to 0.030 part by weight.

5. The composition according to claim 3 wherein amount of sodium carbonate is 0.003 to 0.005 part by weight.

6. The composition according to any one of claims 1 to 5 wherein the composition is a powder.

7. The composition according to claim 6 wherein amount of (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or a pharmaceutically acceptable salt thereof is 50% w/w or more.

8. The composition according to any one of claims 1 to 5 wherein the composition is an aqueous solution.

9. A kit containing (a) a composition comprising 1 part by weight of (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or a pharmaceutically acceptable salt thereof; and (b) a composition comprising 0.002 to 0.040 part by weight of sodium bicarbonate and/or 0.002 to 0.008 part by weight of sodium carbonate.

10. The kit according to claim 9 containing (a) the composition comprising 1 part by weight of (4R,5S,6S)-6-[(1R)-1-hydroxyethyl]-4-methyl-3-{4[(5S)-5-methyl-2,5-dihydro-1H-py-rrol-3-yl]-1,3-thiazol-2-yl}sulfanyl)-7-oxo-1-azabicyclo[3.2.0]hept-2-ene-2-carboxylic acid or a pharmaceutically acceptable salt thereof; and (b) the composition comprising 0.002 to 0.040 part by weight of sodium bicarbonate.

11. The kit according to claim 9 containing (a) the composition comprising 1 part by weight of (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or a pharmaceutically acceptable salt thereof; and (b) the composition comprising 0.002 to 0.008 part by weight of sodium carbonate.

12. The kit according to claim 10 wherein amount of sodium bicarbonate is 0.005 to 0.030 part by weight.

13. The kit according to claim 11 wherein amount of sodium carbonate is 0.003 to 0.005 part by weight.

14. The kit according to any one of claims 9 to 13 wherein the composition (a) is a powder.

15. The kit according to claim 14 wherein amount of the (4R,5S,6S)-6-[(1R)-1-hydroxyethyl]-4-methyl-3-({4-[(5S)-5-methyl-2,5-dihydro-1H-pyrrol-3-yl]-1,8-thiazol-2-yl}sulfanyl)-7-oxo-1-azabicyclo[3.2.0]hept-2-ene-2-carboxylic acid or a pharmaceutically acceptable salt thereof in the composition (a) is 50% w/w or more.

16. The kit according to any one of claims 9 to 13 wherein the composition (a) is an aqueous solution.

17. A package of the kit containing (a) a composition comprising (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}-sulfanyl)-7-oxo-1-azabicyclo[3.2.0]hept-2-ene-2-carboxylic acid or a pharmaceutically acceptable salt thereof; and (b) an instruction indicating to dissolve the composition (a) by using an aqueous solution containing 0.002 to 0.040 part by weight of sodium bicarbonate and/or 0.002 to 0.008 part by weight of sodium carbonate per one part of the (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or a pharmaceutically acceptable salt thereof.

18. The package of the kit according to claim 17 containing (a) the composition comprising (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or a pharmaceutically acceptable salt thereof; and (b) instructions indicating to dissolve the composition (a)by using an aqueous solution containing 0.002 to 0.040 part by weight of sodium bicarbonate per one part of the (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or a pharmaceutically acceptable salt thereof.

19. The package of the kit according to claim 17 containing (a) the composition comprising (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or a pharmaceutically acceptable salt thereof; and (b) an instruction indicating to dissolve the composition (a) by using an aqueous solution containing 0.002 to 0.008 part by weight of sodium carbonate per one part of the (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or a pharmaceutically acceptable salt thereof.

20. The package of the kit according to claim 18 wherein amount of sodium bicarbonate is 0.005 to 0.030 part by weight.

21. The package of the kit according to claim 18 wherein amount of sodium carbonate is 0.003 to 0.005 part by weight.

22. The package of the kit according to any one of claims 17 to 21 wherein the composition (a) is a powder.

23. The package of the kit according to claim 22 wherein amount of the (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or a pharmaceutically acceptable salt thereof in the composition (a) is 50% w/w or more.
